# EUROPEAN PATENT APPLICATION

(11) **EP 3 797 795 A1**
(43) Date of publication of application: **31.03.2021**
(21) Application number: 19807031.0
(22) Date of filing: 20.05.2019
(51) Int. Cl.: A61K 47/68, A61K 47/65, A61P 35/00

(54) **PREPARATION PROCESS OF ANTIBODY DRUG CONJUGATE INTERMEDIATE**

(30) Priority: 21.05.2018 CN 201810487856
(71) Applicant: RemeGen, Ltd., Yantai, Shandong 264006 (CN)
(72) Inventor: HUANG, Changjiang, yantai, Shandong 264006 (CN); YE, Hui, yantai, Shandong 264006 (CN); YAO, Xuejing, yantai, Shandong 264006 (CN); JIE, Haohua, yantai, Shandong 264006 (CN); ZHAI, Shizhong, yantai, Shandong 264006 (CN); FANG, Jianmin, yantai, Shandong 264006 (CN)
(74) Representative: HGF
(86) International application number: PCT/CN2019/087641
(87) International publication number: WO 2019/223653

(57) **Abstract**

The present invention relates to a preparation process of an antibody drug conjugate intermediate. Compared with the prior art, according to the preparation process of the antibody drug conjugate intermediate provided in the present invention, because a drug part participates in the reaction in the last step, loss of the drug part, for example, a fed material such as MMAD/MMAE or MMAF, is reduced, production costs are reduced, and production benefits are increased. In addition, the process provided in the present invention is simple, environment-friendly, and suitable for industrial large-scale popularization.

## Description

### FIELD

The present invention relates to the field of pharmaceutical preparation, specifically to a process for preparing an intermediate of antibody-drug conjugate.

### BACKGROUND

Antibody-drug conjugate (abbreviated as ADC) is a class of anti-tumor drugs consisting of three components: antibody, linker and drug. The number of drugs attached to the antibody (Drug-antibody Ratio, DAR) determines the homogeneity of the antibody-drug conjugate. Excessive number of attached drugs will lead to unstable pharmacodynamics, increased drug metabolism, decreased half-life, and increased systemic toxicity.

The DAR value is primarily determined by the linker. Classical ADCs have two coupling methods: one is amino coupling and the other is thiol coupling, which is currently the most commonly used coupling method. Amino coupling is the coupling of a drug to a lysine (Lys) residue of an antibody via a linker. Since there are 80 lysines on one antibody, 30 of which can be linked, the ADC formed by lysine coupling has a DAR value of 0 to 30, and the product homogeneity is extremely poor. Thiol coupling is to open the interchain disulfide bonds in the antibody first to give free cysteine (Cys) residues, before being coupled to a linker-drug complex capable of pairing with a cysteine residue. Since a single antibody only has 4 pairs of interchain disulfide bonds, 8 free cysteine residues would be generated after the interchain disulfide bonds are all opened. Therefore, the ADCs formed by cysteine residue coupling have DAR values of 0 to 8. Although thiol coupling can better control the number of linkages per antibody, the cleavage of the interchain disulfide bond greatly reduces the stability of the antibody.

Currently, in addition to the classical amino coupling and thiol coupling, there is a new coupling method: bridging coupling. The bridging coupling method is a new coupling method developed based on thiol coupling, in which the linker is simultaneously coupled to two free thiol groups on the antibody, and the DAR value of the antibody-drug conjugate produced in this manner is 0 to 4. A variety of linkers capable of being covalently linked to an antibody by way of bridging coupling are disclosed in a Chinese patent publication CN107921030A, which discloses an intermediate of antibody-drug conjugate (Py-MAA-Val-Cit-PAB-MMAE) (wherein Py is triacryloylhexahydro triazine, its CAS is 959-52-4, and is available from J&K Scientific and Nanjing Chemlin Chemical Industry Co., Ltd.) as shown below on page 42 of the specification.

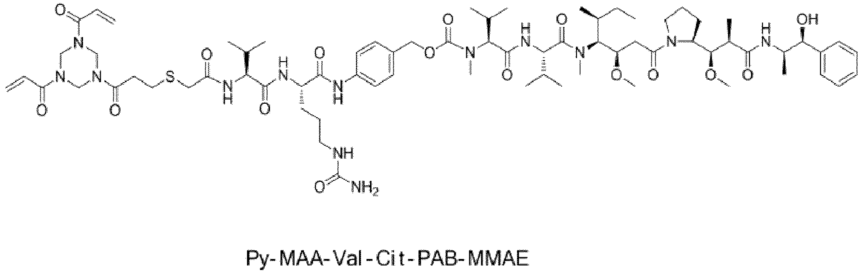

In addition, a process for preparing an intermediate of antibody-drug conjugate (Py-MAA-Val-Cit-PAB-MMAD) in which the Drug is MMAD (Demethyldolastatin) is also disclosed on page 32 of the patent application:

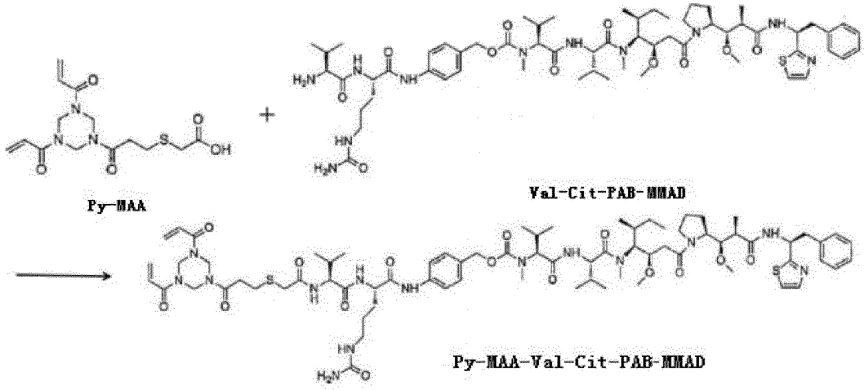

The process involves first coupling Val-Cit-PAB with Drug (MMAD) to form a Val-Cit-PAB-MMAD conjugate, and then reacting with Py-MAA to form an antibody-drug conjugate intermediate Py-MAA-Val-Cit-PAB-MMAD. Due to high price for the drug (such as MMAD/MMAE or MMAF, etc.) used in the antibody-drug conjugate and great feed loss for the production of drug using the above-mentioned process, the production cost is high, making it not suitable for industrial mass production.

### SUMMARY

The present disclosure provides a process for preparing an intermediate of antibody-drug conjugate, specifically provides a process for preparing an intermediate of antibody-drug conjugate comprising a linker and a drug moiety. The intermediate of antibody-drug conjugate is Py-MAA-Val-Cit-PAB-D, in which Py-MAA-Val-Cit-PAB is the linker, and D represents the attached drug moiety. The process is characterized in that it comprises the following reactions:

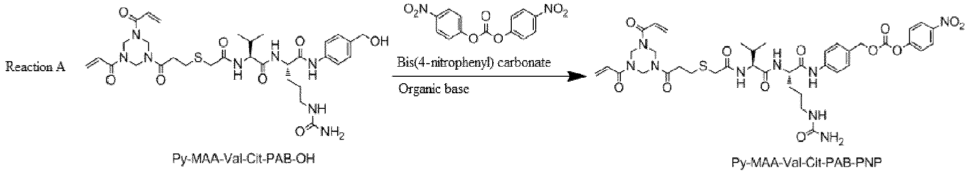

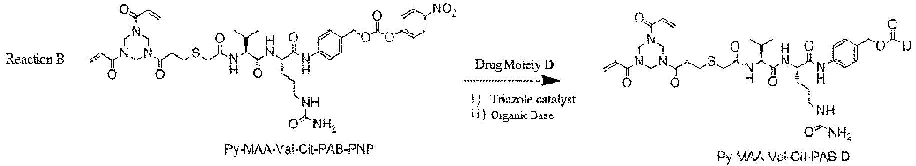

Further, the preparation process comprises the following reaction procedure:
Reaction A: dissolving Py-MAA-Val-Cit-PAB-OH in a solvent, adding bis(4-nitrophenyl) carbonate, stirring until dissolved or the solution is slightly turbid, and then adding an organic base;
Reaction B: after completion of Reaction A, adding a triazole catalyst, the drug moiety D, and an organic base, and after completion of the reaction, carrying out purification to obtain Py-MAA-Val-Cit-PAB-D. Preferably, pre-HPLC is used for the purification.

Further, the drug moiety D is an auristatin class cytotoxic agent, an anthramycin class cytotoxic agent, an anthracycline class cytotoxic agent or a puromycin class cytotoxic agent.

Preferably, the auristatin class cytotoxic agent is selected from the group consisting of MMAE, MMAF, MMAD and a derivative thereof; the anthramycin class cytotoxic agent is anthramycin; the anthracycline class cytotoxic agent is selected from the group consisting of daunorubicin, doxorubicin, epirubicin, idarubicin, valrubicin and mitoxantrone; and the puromycin class cytotoxic agent is puromycin .

Preferably, the Py-MAA-Val-Cit-PAB-D has the structures as represented by Formulas (1-11):

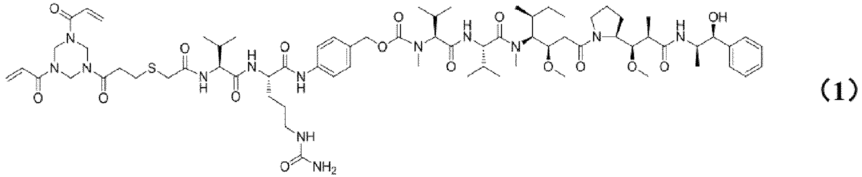

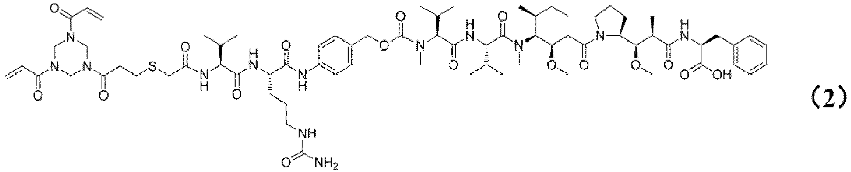

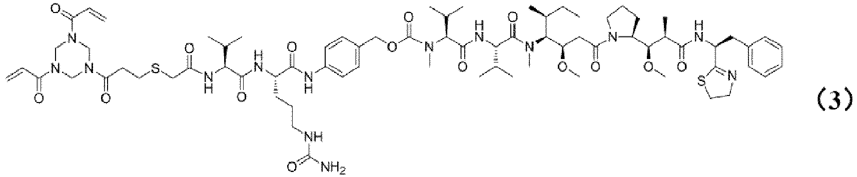

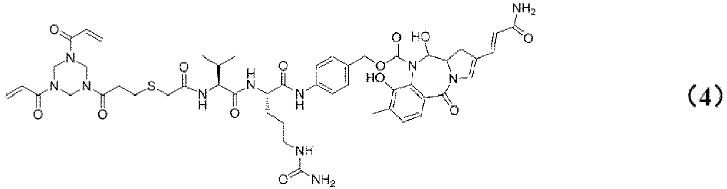

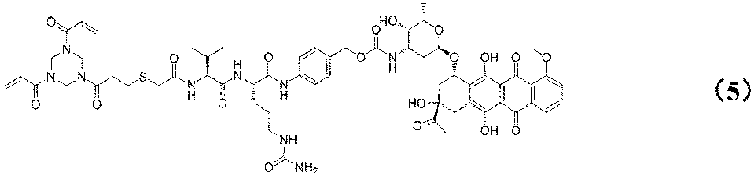

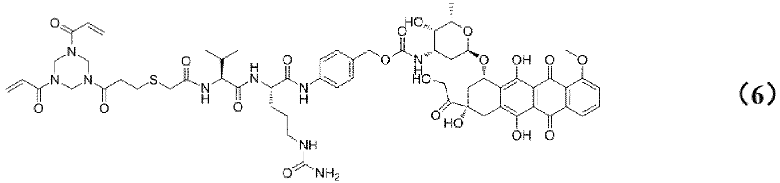

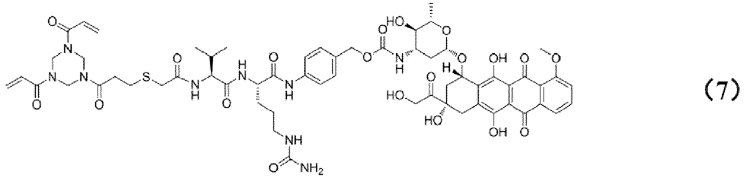

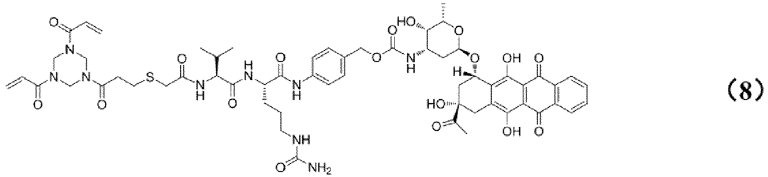

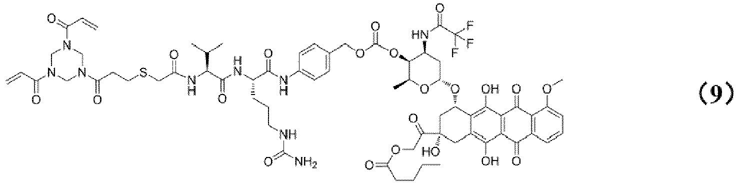

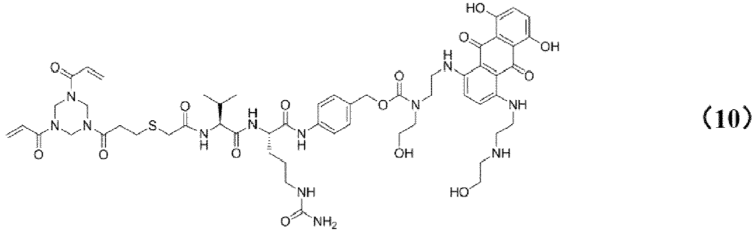

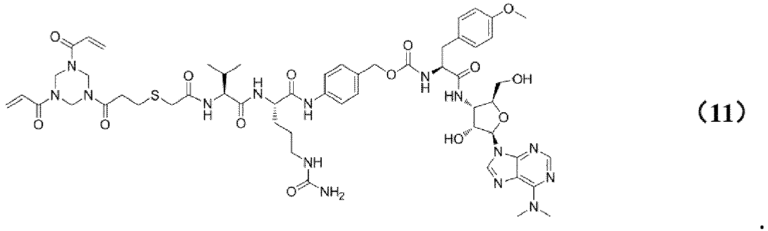

Further, the solvent is a polar solvent and/or a non-polar solvent. The polar solvent is one or more selected from the group consisting of DMF, DMA and NMP; the non-polar solvent is one or more selected from the group consisting of dichloromethane and carbon tetrachloride.

Further, the organic base is one or more selected from the group consisting of N,N-diisopropylethylamine, triethylamine and pyridine, preferably, one or two selected from the group consisting of N,N-diisopropylethylamine and pyridine.

Further, the triazole catalyst is one or more selected from the group consisting of 1-hydroxybenzotriazole, 1-hydroxy-7-azabenzotriazole, ethyl 1-hydroxy-1H-1,2,3 -triazole-4-carboxylate, preferably 1-hydroxybenzotriazole.

Further, the reaction temperature of the Reaction A is 5 to 35 °C, and the reaction duration is 2 to 10 hours.

Preferably, in the Reaction A, controlling the temperature of the reaction system to be in a range from 5 to 15 °C prior to the addition of bis(4-nitrophenyl) carbonate, and further preferably 10 °C.

Preferably, in the Reaction A, controlling the temperature of the reaction system to be in a range from 20 to 35 °C after the addition of the organic base, preferably 30 °C.

Further, the reaction temperature of the Reaction B ranges from 5 to 30 °C, and the reaction duration ranges from 15 to 48 hours.

Preferably, in the Reaction B, controlling the temperature of the reaction system to be in a range from 5 to 15 °C prior to the addition of the triazole catalyst, preferably 10 °C.

Preferably, in the Reaction B, controlling the temperature of the reaction system to be in a range from 15 to 30 °C after the addition of the organic base, preferably 20 °C. Further, the molar ratio of Py-MAA-Val-Cit-PAB-OH, bis(4-nitrophenyl) carbonate and the organic base in Reaction A is 1: 1-5: 1-3.

Further, the molar ratio of the triazole catalyst, MMAE, the organic base and Py-MAA-Val-Cit-PAB-OH in the Reaction B is 1-3: 1-3 : 1.5-30 :1.

Further, the present disclosure also provides using any one of the above-mentioned preparation processes to prepare an intermediate of an antibody-drug conjugate, which is Py-MAA-Val-Cit-PAB-MMAF, and its structure is as represented by Formula (2):

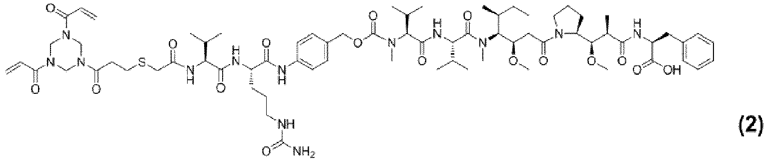

Further, the present disclosure also provides using any one of the above-mentioned preparation processes to prepare an intermediate of antibody-drug conjugate, which is Py-MAA-Val-Cit-PAB-MMAD, and its structure is as represented by Formula (3):

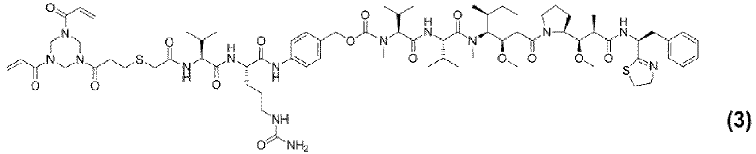

Further, the present disclosure also provides using any one of the above-mentioned preparation processes to prepare an intermediate of antibody-drug conjugate, which has a structure as represented by Formula (4):

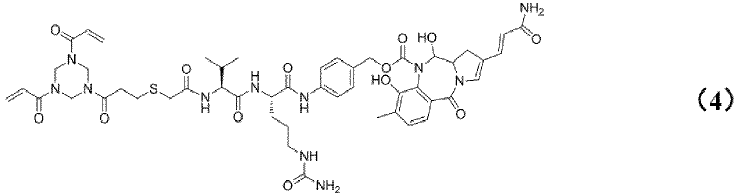

Further, the present disclosure also provides using any one of the above-mentioned preparation processes to prepare an intermediate of antibody-drug conjugate, which has a structure as represented by Formula (5):

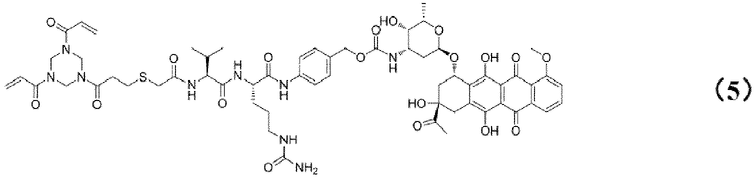

Further, the present disclosure also provides using any one of the above-mentioned preparation processes to prepare an intermediate of antibody-drug conjugate, which has a structure as represented by Formula (6):

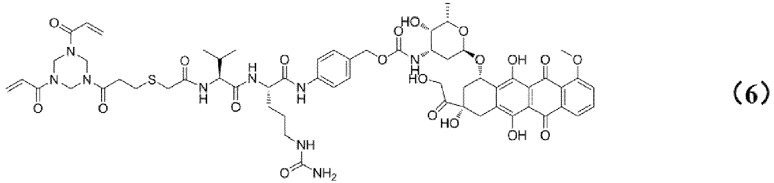

Further, the present disclosure also provides using any one of the above-mentioned preparation processes to prepare an intermediate of antibody-drug conjugate, which has a structure as represented by Formula (7):

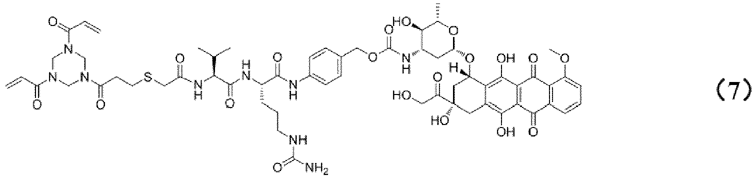

Further, the present disclosure also provides using any one of the above-mentioned preparation processes to prepare an intermediate of antibody-drug conjugate, which has a structure as represented by Formula (8):

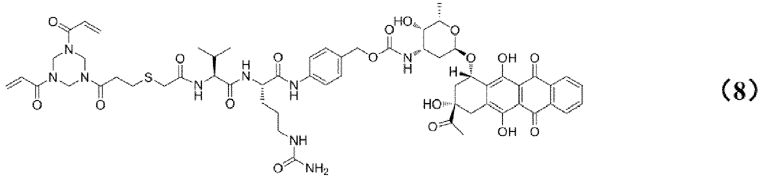

Further, the present disclosure also provides using any one of the above-mentioned preparation processes to prepare an intermediate of antibody-drug conjugate, which has a structure as represented by Formula (9):

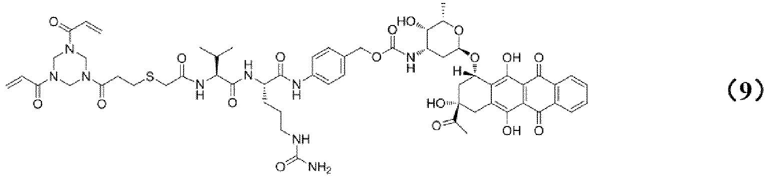

Further, the present disclosure also provides using any one of the above-mentioned preparation processes to prepare an intermediate of antibody-drug conjugate, which has a structure as represented by Formula (10):

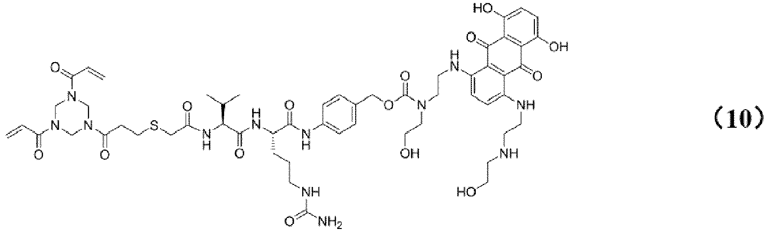

Further, the present disclosure also provides using any one of the above-mentioned preparation processes to prepare an intermediate of antibody-drug conjugate, which has a structure as represented by Formula (11):

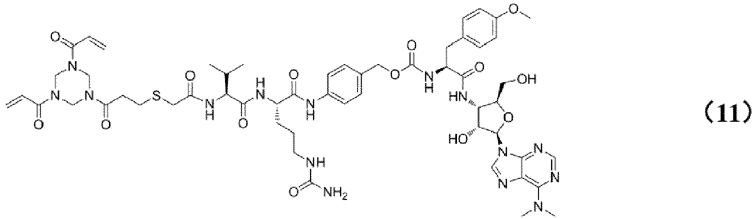

In some particular embodiments, the process comprises the following steps:
Reaction A: dissolving Py-MAA-Val-Cit-PAB in DMF, adding bis(p-nitrophenyl)carbonate, stirring until they are dissolved or slightly turbid before adding N,N-diisopropylethylamine dropwise, separating and purifying to obtain Py-MAA-Val-Cit-PAB-PNP;
Reaction B: dissolving Py-MAA-Val-Cit-PAB-PNP in DMF, adding 1-hydroxybenzotriazole, MMAE, N,N-diisopropylethylamine and pyridine, carrying out pre-HPLC purification after completion of the reaction to obtain compound Py-MAA-Val-Cit-PAB-MMAE as represented by Formula (1).
wherein the structure of the Py-MAA-Val-Cit-PAB-PNP is shown as follow:

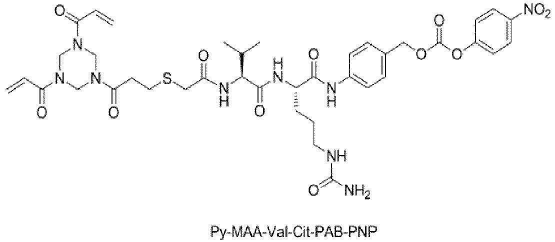

Furthermore, the separation and purification in the above-mentioned reaction process may be as follows:
(1) centrifuging the reaction solution of Reaction A to obtain a supernatant;
(2) adding solvent 1 to the supernatant, and stirring well;
(3) adding solvent 2 dropwise at 0 to 10 °C, and stirring well;
(4) filtering under suction to obtain a filter cake;
(5) re-dissolving the filter cake in a mixed solution of solvent 3 and solvent 4;
(6) adding the resultant dropwise to solvent 5 at 0 to 10 °C, and stirring;
(7) filtering under suction to obtain an off-white powder, which is purified Py-MAA-Val-Cit-PAB-PNP.

Thereinto, the solvent 1 is a moderately polar solvent, preferably selected from the group consisting of ethyl acetate, dichloromethane, methanol, methyl tert-butyl ether and the like, most preferably ethyl acetate;
the solvent 2 is a weakly polar solvent, preferably selected from the group consisting of petroleum ether, n-hexane, n-pentane, cyclohexane and the like, most preferably petroleum ether;
the solvents 3 and 4 are solvents in which Py-MAA-Val-Cit-PAB-PNP is easy to dissolve, preferably selected from the group consisting of acetic acid, trifluoroacetic acid, formic acid, methanol, ethanol and the like, most preferably acetic acid and methanol;
the solvent 5 is a solvent in which Py-MAA-Val-Cit-PAB-PNP is poorly soluble, preferably selected from the group consisting of acetonitrile, ethyl acetate, dichloromethane, water and the like, most preferably water.

The volume/weight ratio of the solvent 1, the solvent 2, the solvent 3 and the solvent 4 and Py-MAA-Val-Cit-PAB is 40-100: 80-200: 5-15: 1-3: 15-30: 1 .

In some particular embodiments, the above-mentioned separation and purification may be as follows:
(1) centrifuging the intermediate product A to obtain a supernatant;
(2) adding ethyl acetate to the supernatant, and stirring well;
(3) adding petroleum ether dropwise at 0 to 10 °C, and stirring well;
(4) filtering under suction, and taking the filter cake;
(5) re-dissolving the filter cake in a mixed solution of acetic acid and methanol;
(6) adding purified water dropwise at 0 to 10 °C, and stirring;
(7) filtering under suction to obtain a off-white powder, which is purified Py-MAA-Val-Cit-PAB-PNP.

Compared with the process for preparing an intermediate of the antibody-drug conjugate (Py-MAA-Val-Cit-PAB-MMAD) disclosed by the Chinese patent publication CN107921030A on page 43 of the specification, the process for preparing an intermediate of the antibody-drug conjugate provided by the present disclosure significantly reduces the feed loss for drug moiety (such as MMAD/MMAE or MMAF, etc.) as drug moiety is involved in the last step of the reaction, thereby effectively reducing production costs, as well as increasing production efficiency. In addition, the process provided by the present disclosure is simple, environmentally friendly and suitable for large-scale industrialization.

### DETAILED DESCRIPTION

### Abbreviations

| Abbreviations | Structure / English name |
|---|---|
| Py | 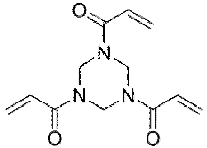 |
| | Triacryloylhexahydro triazine |
| Py-MAA | 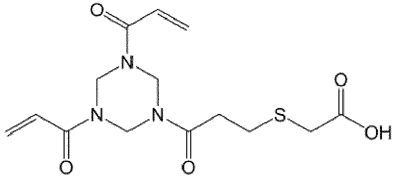 |
| | Triacryloylhexahydro triazine-mercaptoacetic acid |
| Py-MAA- Val-Cit-PAB-OH | 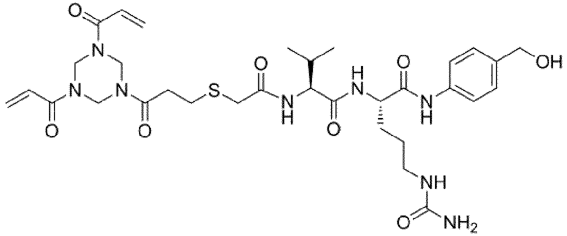 |
| | Triacryloylhexahydro triazine-mercaptoacetic acid-valine-citrulline-p-aminobenzyl alcohol |
| Py-MAA- Val-Cit-PAB-PNP | 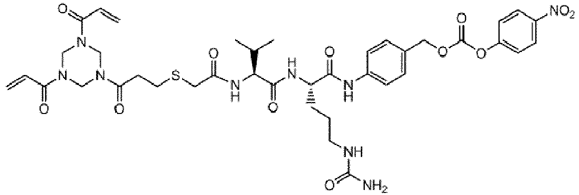 |
| | Triacryloylhexahydro triazine-mercaptoacetic acid- valine-citrulline-p-aminobenzyloxycarbonyl-p-nitrophenyl ester |
| Py-MAA- Val-Cit-PAB-MMAE | 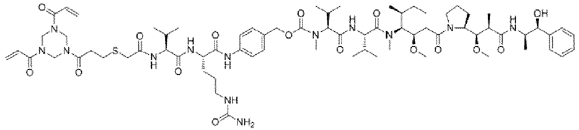 |
| | Triacryloylhexahydro triazine-mercaptoacetic acid-valine-citrulline-p-aminobenzyloxycarbonyl Monomethyl auristatin E |
| MMAE | 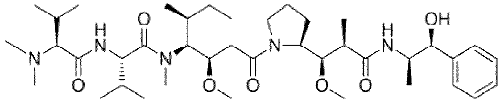 |
| MMAD | 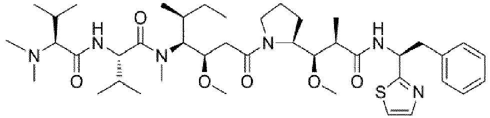 |
| MMAF | 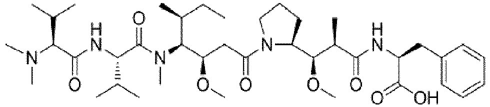 |
| NPC | 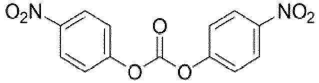 |
| | Bis(4-nitrophenyl) carbonate |
| Anthramycin | 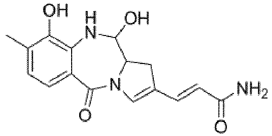 |
| Daunorubicin | 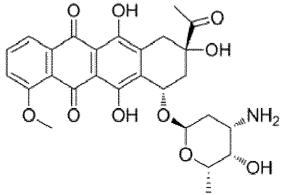 |
| Doxorubicin | 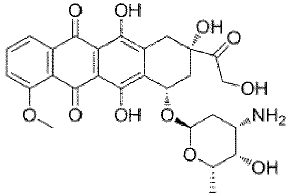 |
| Epirubicin | 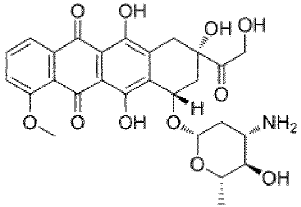 |
| Idarubicin | 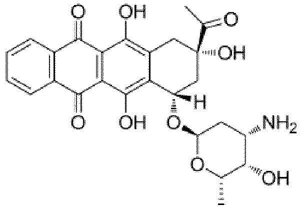 |
| Valrubicin | 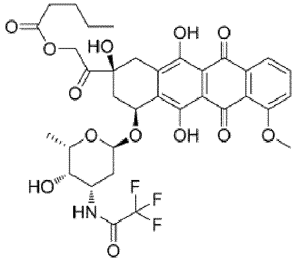 |
| Mitoxantrone | 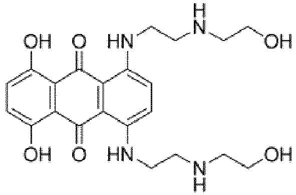 |
| puromycin | 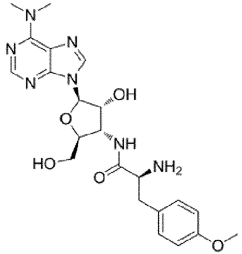 |

### Definitions

Unless otherwise defined, all technical and scientific terms used herein have the same meaning as interpreted by a person having ordinary skill in the art.

Although numerical ranges and parameter approximations are shown in a broad scope in the present disclosure, the numerical values shown in specific embodiments are described as being as accurate as possible. However, any numerical value inherently contains certain errors due to the standard deviations present in their respective measurements. In addition, all ranges disclosed herein are to be interpreted as encompassing any and all sub-ranges. For example, the stated range of "1 to 10" should be considered to contain any and all subranges between the minimum 1 and the maximum 10 (including the endpoints); that is all subranges starting with a minimum of 1 or greater, such as 1 to 6.1, and subranges ending with a maximum of 10 or less, such as 5.5 to 10. In addition, any reference that is referred to as "incorporated herein" is interpreted to be incorporated in its entirety.

In addition, it should be noted that, as used in the specification, the singular forms include the plural forms of the subject matter, unless clearly and explicitly limited to one subject matter. The term "or" can be used interchangeably with the term "and/or" unless the context clearly dictates otherwise.

The term "antibody-drug conjugate" as used in the present disclosure represents a compound in which an antibody/antibody functional fragment, a linker, and a drug moiety are chemically linked together, and the structure thereof usually consists of three parts: an antibody or antibody ligand, a drug moiety, and a linker that couples the antibody or antibody ligand and the drug. Currently the preparation of antibody-drug conjugates is usually divided into two steps: the first step is to chemically react a linker with a drug moiety to form a "linker-drug" conjugate, and the second step is to covalently couple the linker part of the "linker-drug" conjugate to an antibody/antibody functional fragment via a thiol or amino group. The term "intermediate of antibody-drug conjugate" as used in the present disclosure refers to the "linker-drug" conjugate as described above, and further, the "intermediate of antibody-drug conjugate" referred to in the present disclosure generally refers to those "linker-drug" conjugate in which a linker and a drug are coupled together by a "-CO-NH-" bond via amine transesterification.

The terms "linker" and "linker part" as used in the present disclosure refers to the part of an antibody-drug conjugate that links an antibody to a drug, which may be cleavable or non-cleavable. A cleavable linker (i.e., a linker which is cleavable or a biodegradable linker) can be cleaved inside or on the target cell so as to release the drug. In some embodiments, the linker of the present disclosure is selected from cleavable linkers, such as a disulfide-based linker (which is selectively cleaved in tumor cells which have higher concentration of sulfhydryl groups), a peptide linker (which is cleaved by an enzyme in tumor cells), or a hydrazone linker. In another embodiments, the linker of the present disclosure is selected from non-cleavable linkers (i.e. a linker which is not cleavable), such as a thioether linker. In another embodiments, the linker of the present disclosure is a combination of a cleavable linker and a non-cleavable linker.

The terms "drug" and "drug moiety" as used in the present disclosure generally refers to any compound having desired biological activity and reactive functional group for the preparation of the conjugates of the present disclosure. The desired biological activity includes diagnosing, curing, alleviating, treating, preventing diseases in humans or other animals. As novel drugs are continuously discovered and developed, these novel drugs should also be included in the drugs of the present disclosure. Specifically, the drug includes, but is not limited to, cytotoxic drugs, cell differentiation factors, stem cell trophic factors, steroid drugs, drugs for treating autoimmune diseases, anti-inflammatory drugs, or drugs for infectious diseases. More specifically, the drug includes, but is not limited to, tubulin inhibitors or DNA, RNA damaging agents.

### Examples

The followings are examples of the methods and compositions of the present disclosure. It should be understood that various other embodiments may be implemented in light of the above definitions and the general description.

### Example 1 Preparation of Py-MAA

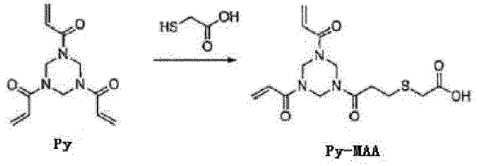

Compound Py (1.87 g, 7.51 mmol) (CAS: 959-52-4) and Et₃N (104 µL, 0.75 mmol) were dissolved in anhydrous CH₂Cl₂ (40 mL), a solution of thioglycolic acid (103.9 µL, 1.50 mmol) in CH₂Cl₂ (40 mL) was added dropwise. After completion of the addition, the temperature of the system was raised to room temperature and it was stirred overnight. After completion of the reaction, the solvent was removed in vacuo, and the crude product was purified by column chromatography to obtain white solid Py-MAA (1.87 g, 40.1%).

### Example 2 Comparative Example: Preparation of Py-MAA-Val-Cit-PAB-MMAE (which is the process disclosed on page 32 of the specification of the Chinese patent publication CN107921030A).

The procedure and method are shown as below:

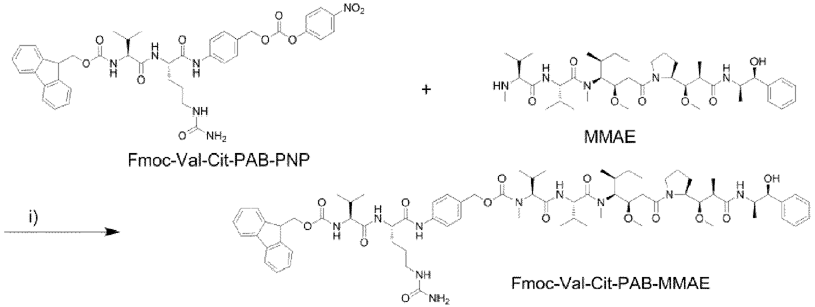

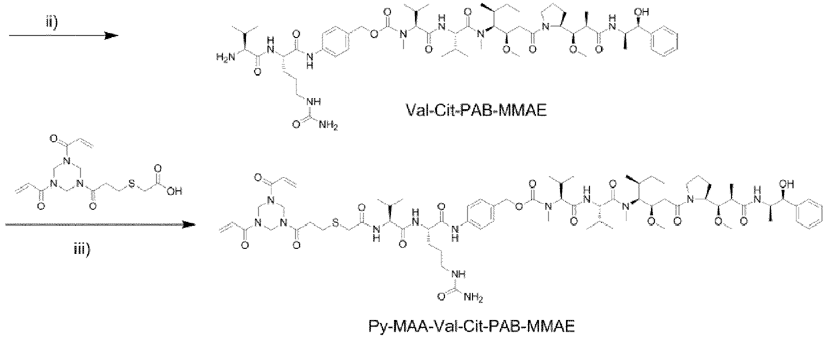

### (1) Preparation of Fmoc-Val-Cit-PAB-MMAE

Fmoc-Val-Cit-PAB-PNP (388.4 mg, 0.507 mmol) was taken and dissolved in DMF (40 m) under the protection of nitrogen at 0 °C, and HOBt (68.4 mg, 0.507 mmol) and compound MMAE (400.0 mg, 0.558 mmol) were added. After 15 minutes, pyridine (8 ml) and DIPEA (106.2 µL, 0.608 mmol) were added and reacted at 0 °C for 30 minutes. The temperature was increased to room temperature and it was stirred for 3 hours. The reaction mixture was supplemented with DIPEA (106.2 µL, 0.608 mmol) and the reaction was continued at room temperature for 24 hours. After completion of the reaction, the mixture was concentrated in vacuo. The crude product was purified by column chromatography to obtain a white solid compound Fmoc-Val-Cit-PAB-MMAE (325 mg, yield 47.7%).

### (2) Preparation of Val-Cit-PAB-MMAE

Compound Fmoc-Val-Cit-PAB-MMAE (325 mg, 0.289 mmol) was dissolved in DMF (6 mL), before diethylamine (3 mL) was added, and the reaction was continued with stirring for 2.5 hour. After completion of the reaction, the solvent was removed under reduced pressure to obtain a crude product Val-Cit-PAB-MMAE (220 mg, yield: 67.8%), which can be used for the next reaction directly without purification.

### 3) Synthesis of Py-MAA-Val-Cit-PAB-MMAE

Compound Py-MAA (80.2 mg, 0.235 mmol), HOBt (31.8 mg, 0.235 mmol) and DIC (29.6 mg, 0.235 mmol) were taken and dissolved in DMF (10 mL) and cooled to 0 °C in an ice bath. Compound Val-Cit-PAB-MMAE (220.0 mg, 0.196 mmol) and DIPEA (25.3 mg, 0.196 mmol) were added to the reaction solution, the temperature was raised to room temperature and the reaction was performed for 24 hours. After completion of the reaction, the crude product was purified by column chromatography (methanol: dichloromethane = 60:1- 30:1) to obtain a white solid compound Py-MAA-Val-Cit-PAB-MMAE (147.4 mg, yield 52%). LCMS m/z(ES+), 1445 .78 (M+H)+.

Py-MAA-Val-Cit-PAB-MMAE was prepared by the process for preparing Py-MAA-Val-Cit-PAB-MMAD as disclosed on page 32 of the specification of Chinese patent publication CN107921030A. The feed amount of MMAE was 400 mg, and the obtained Py-MAA-Val-Cit-PAB-MMAE was 147.4 mg.

### Example 3 Improved Preparation Method of Py-MAA-Val-Cit-PAB-MMAE

### (1) Preparation of Py-MAA-Val-Cit-PAB

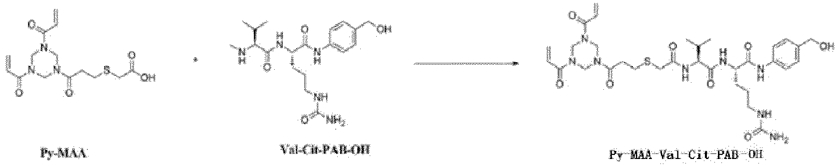

Compound Py-MAA (10.00 g, 29.33 mmol) was put in tetrahydrofuran (200 mL), before N,N'-Carbonyldiimidazole (7.13 g, 44.00 mmol) and Val-Cit-PAB-OH (13.34 g, 35.20 mmol) were added, and stirred at room temperature for 24 hours. Petroleum ether (200 mL) was added, stirred for 0.5 hours, and filtered to obtain a white solid. The white solid was purified by preparative high performance liquid chromatography, the resultant was subjected to rotary evaporation to obtain Py-MAA-Val-Cit-PAB-OH (6.67 g, 32.4%, a white solid powder).

### (2) Preparation of Py-MAA-Val-Cit-PAB-PNP

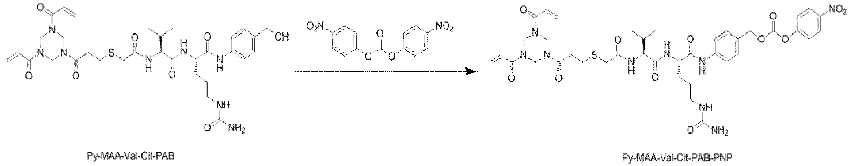

Py-MAA-Val-Cit-PAB-OH (10.00 g, 1.0 eq) and DMF (V_{DMF}/Wp_{y-MAA-Val-Cit-PAB} = 15-25 mL/g) were added into a three neck flask under the protection of nitrogen, stirred under the protection of nitrogen until the solid was dissolved or the solution was slightly turbid. The temperature was reduced to 10 °C for the three neck flask, NPC (1.5-2.5 eq, 6.50-10.83 g) solid was added in one portion and stirred until dissolved or until the solution was slightly turbid. DIPEA (1.0-1.5 eq, 1.84-2.76 g) was added dropwise under the protection of nitrogen. The temperature was raised to 30 °C, and the reaction was monitored by UPLC until the purity of Py-MAA-Val-Cit-PAB was less than 5.0%, then the reaction was terminated.

The reaction solution was centrifuged, and the supernatant was poured into a beaker of suitable size, into which EA (V_{EA}/W_{Py-MAA-Val-Cit-PAB} = 60 ± 2) was added and stirred with an electric mixer; PE (V_{PE}/W_{Py-MAA-Val-Cit-PAB} = 120 ± 2) was added dropwise at 0 to 6 °C, and stirred for 20 to 30 minutes after dropwise addition. After suction filtration, the filter cake was taken, and dissolved with acetic acid (V_{AcOH}/W_{Py-MAA-Val-Cit-PAB}=7) and methanol (V_{MeOH}/W_{Py-MAA-Val-Cit-PAB}) to obtain a solution.

Purified water (V_{Water}/W_{Py-MAA-Val-Cit-PAB} = 21) was poured into a beaker of suitable size, mechanically stirred, and cooled to about 3 °C. The above-mentioned solution was added dropwise to purified water and stirred for 15 minutes after the dropwise addition. After suction filtration, the filter cake was obtained and lyophilized to give Py-MAA-Val-Cit-PAB-PNP (a white to off-white powder, 10.69 g, yield 86.54%).

### (3) Preparation of Py-MAA-Val-Cit-PAB-MMAE

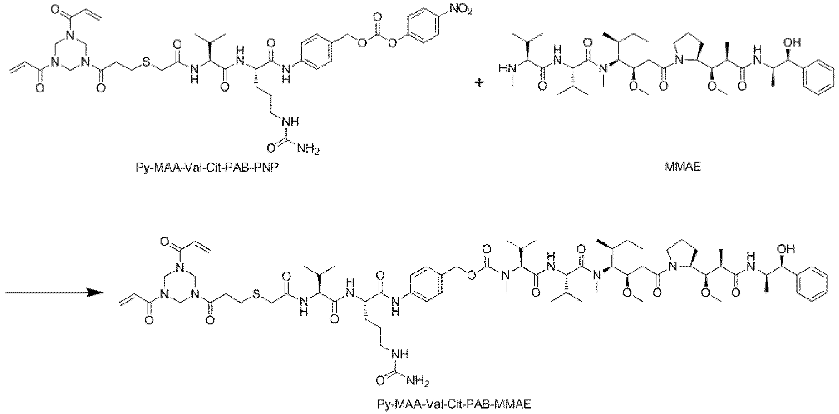

Py-MAA-Val-Cit-PAB-PNP (9.00 g, 1.0 eq) and DMF (V_{DMF}/W_{Py-MAA-Val-Cit-PAB-PNP} = 15.0-25.0 mL/g) were added into a reaction vessel, stirred under the protection of nitrogen until clear or slightly turbid. The internal temperature was maintained at 10 °C, while HOBt (2.10 g, 1.5 eq) was added and stirred for 20 minutes. MMAE (8.93 g, 1.2 eq) was added, and the stirring was continued for 20 minutes. A mixed solution of DIPEA (1.34 g, 1.0 eq) and pyridine (V_{pyridine}/V_{DIPEA} = 5) was added dropwise, and the temperature was raised to maintain the internal temperature at 20 °C. After 36 hours of reaction, UPLC monitoring was performed once every other hour, and when the purity of MMAE in the reaction solution was < 2.00%, the reaction was terminated.

A suitable sized beaker was taken, into which a saturated aqueous sodium chloride solution (referred to as brine, V_{brine}/V_{reaction} solution = 10) was added, and the temperature was reduced until the internal temperature was 5 °C. The reaction solution was added dropwise to saturated sodium chloride solution. A crude product was obtained by centrifugation, and purified using preparative high performance liquid chromatography. After lyophilization, Py-MAA-Val-Cit-PAB-MMAE (a white powder, 9.79 g, yield 65.27%) was obtained.

In the present example, the feed amount of MMAE was 8.93 g, with the obtained amount of Py-MAA-Val-Cit-PAB-MMAE being 9.79 g.

Comparing the processes provided in Example 2 and Example 3 in terms of the feed amount of MMAE and the mass of the final product (the comparison results are shown in Table 1), it can be seen from Table 1 that the final obtained amount of Py-MAA-Val-Cit-PAB-MMAE prepared by the preparation process of Example 2 was 147.4 mg while the feed amount of MMAE was 400 mg; the final obtained amount of Py-MAA-Val-Cit-PAB-MMAE prepared by the preparation process of Example 3 was 9.79 g while the feed amount of MMAE was 8.93 g.

**Table 1 Comparison of Feed Amount of MMAE and Obtained Amount of Final Product**

| Examples | Feed Amount of MMAE | Final Obtained Amount of Py-MAA-Val-C it-PAB-MMAE | Input-output Ratio of MMAE (Feed Amount of MMA/ Final Obtained Amount of Py-MAA-Val-Cit-PAB-MMAE | Percentage Reduction in Feed Amount compared with Comparative Example (Example 2) |
|---|---|---|---|---|
| Example 2 | 400 mg | 147.4 mg | 2.71 | - |
| Example 3 | 8.93 g | 9.79 g | 0.91 | 66.4% |

The input-output rate of MMAE represents how many units of MMAE are required to be fed in order to obtain each unit mass of Py-MAA-Val-Cit-PAB-MMAE. As can be seen from Table 1, by using the method of Example 2, 2.71 unit mass of MMAE is required to obtain 1 unit mass of Py-MAA-Val-Cit-PAB-MMAE; while by adopting the method of Example 3, only 0.91 unit mass of MMAE is required to be fed. The feed amount reduced by 66.4%, which significantly reduces production costs.

The above described is merely a preferred embodiment, which is only illustrative and not limiting the combinations of features necessary to practice the invention. The provided title is not to limit the various embodiments of the invention. Terms such as "comprising" and "including" are not intended to limit. In addition, unless otherwise indicated, plural forms is included when there is no numeral modification, and "or" means "and/or". Unless otherwise defined herein, the meaning of all technical and scientific terms used herein is the same as what is commonly interpreted by a person having ordinary skill in the art.

All publications and patents mentioned in this application are hereby incorporated by reference. Numerous modifications and variations of the described methods and compositions of the present invention are obvious to a person having ordinary skill in the art. Although the invention has been described by way of specific preferred embodiments, it should be understood that the invention as claimed should not be unduly limited to these specific embodiments. In fact, those numerous modifications and variations used to practice the modes of the present invention, which are obvious to a person having ordinary skill in the art, are intended to be included within the scope of the appended claims.

## Claims

1. A process for preparing an intermediate of antibody-drug conjugate comprising a linker and a drug moiety, in which the intermediate of antibody-drug conjugate is Py-MAA-Val-Cit-PAB-D, the Py-MAA-Val-Cit-PAB in the intermediate is the linker and the D in the intermediate represents the attached drug moiety, and the attached drug moiety contains free amino group, wherein it comprises the following reactions:

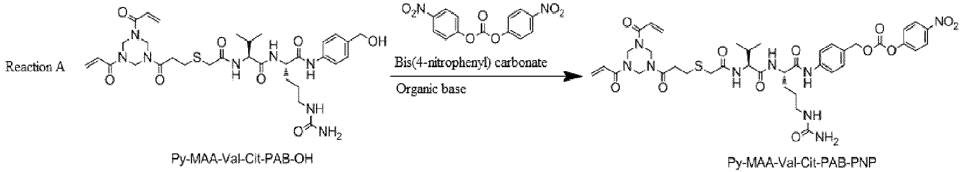

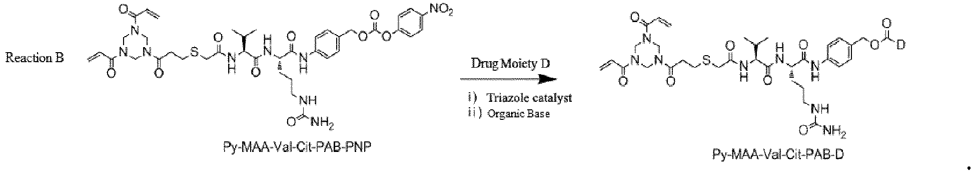

2. The process according to claim 1, wherein
Reaction A: dissolving Py-MAA-Val-Cit-PAB-OH in a solvent, adding bis(4-nitrophenyl) carbonate, stirring until dissolved or the solution is slightly turbid, and then adding an organic base;
Reaction B: after completion of Reaction A, adding a triazole catalyst, the drug moiety D and an organic base, and after completion of the reaction, carrying out purification to obtain Py-MAA-Val-Cit-PAB-D.

3. The process according to claim 1 or 2, wherein the drug moiety D is an auristatin class cytotoxic agent, an anthramycin class cytotoxic agent, an anthracycline class cytotoxic agent or a puromycin class cytotoxic agent.

4. The process according to claim 3, wherein the auristatin class cytotoxic agent is selected from the group consisiting of MMAE, MMAF, MMAD and a derivative thereof; the anthramycin class cytotoxic agent is anthramycin or a derivative thereof; the anthracycline class cytotoxic agent is selected from the group consisiting of daunorubicin, doxorubicin, epirubicin, idarubicin, valrubicin, mitoxantrone and a derivative thereof; and the puromycin class cytotoxic agent is puromycin or a derivative thereof.

5. The process according to claim 4, wherein the structure of the Py-MAA-Val-Cit-PAB-D is represented by Formulas (1-11):

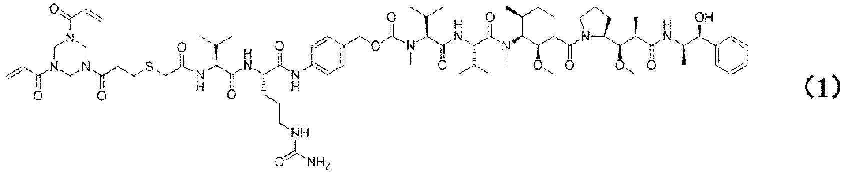

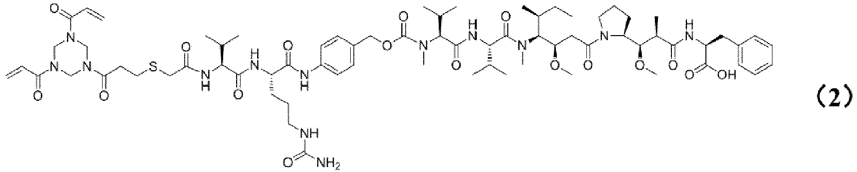

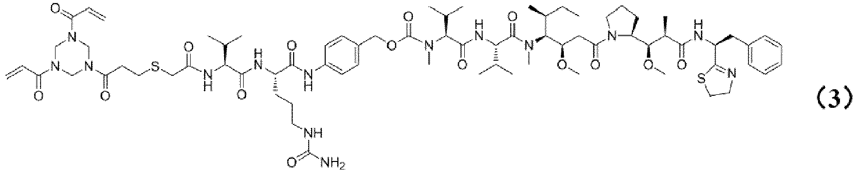

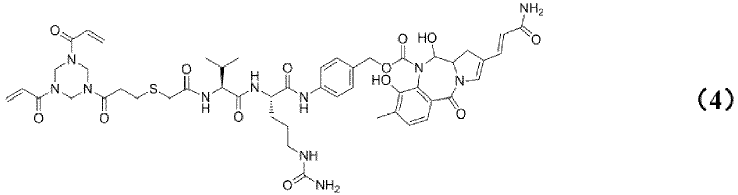

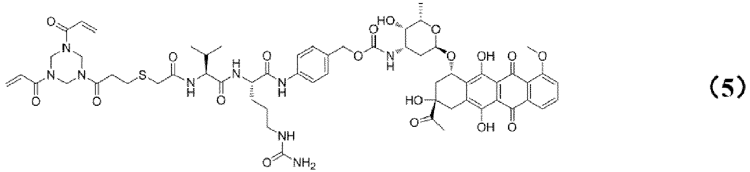

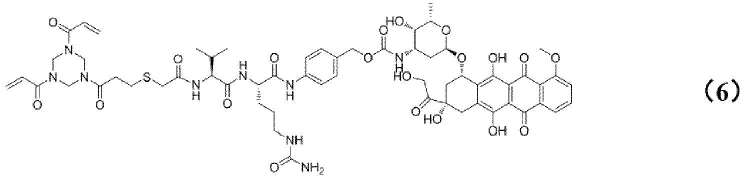

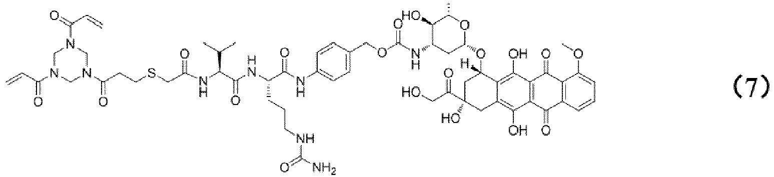

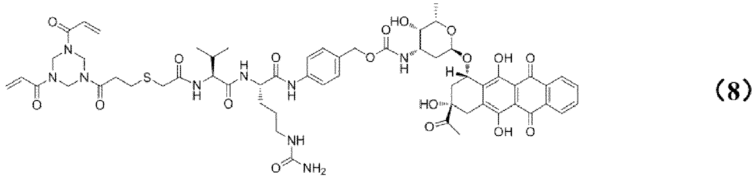

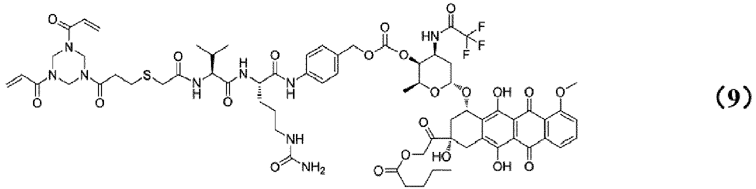

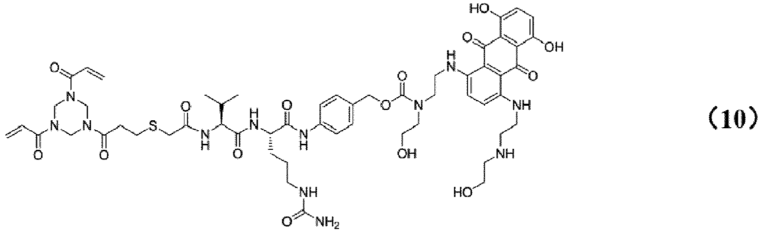

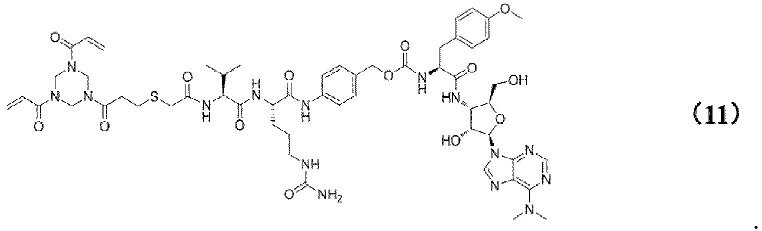

6. The process according to any one of the preceding claims, wherein the solvent is a polar solvent and/or a non-polar solvent, the polar solvent is one or more selected from the group consisting of DMF, DMA and NMP; the non-polar solvent is one or more selected from the group consisting of dichloromethane and carbon tetrachloride.

7. The process according to any one of the preceding claims, wherein the organic base is one or more selected from the group consisting of N,N-diisopropylethylamine, triethylamine and pyridine, preferably one or two selected from the group consisting of N,N-diisopropylethylamine and pyridine.

8. The process according to any one of the preceding claims, wherein the triazole catalyst is one or more selected from the group consisting of 1-hydroxybenzotriazole, 1-hydroxy-7-azabenzotriazole and ethyl 1-hydroxy-1H-1,2,3-triazole-4-carboxylate, preferably 1-hydroxybenzotriazole.

9. The process according to any one of the preceding claims, comprising: in the Reaction A, controlling the temperature of the reaction system to be in the range from 5 to 15 °C prior to the addition of bis(4-nitrophenyl) carbonate, preferably 10 °C; and further preferably, in the Reaction A, controlling the temperature of the reaction system to be in the range from 20 to 35 °C after addition of the organic base, preferably 30 °C.

10. The process according to any one of the preceding claims, comprising: in the Reaction B, controlling the temperature of the reaction system to be in the range from 5 to 15 °C prior to the addition of the triazole catalyst, preferably 10 °C; further preferably, in the Reaction B, controlling the temperature of the reaction system to be in the range from 15 to 30 °C after the addition of the organic base, preferably 20 °C.

11. The preparation process according to any one of the preceding claims, wherein, in the Reaction A, the molar ratio of Py-MAA-Val-Cit-PAB-OH. bis(4-nitrophenyl) carbonate and the organic base is 1: 1-5: 1-3.

12. The process according to any one of the preceding claims, wherein, in the Reaction B, the molar ratio of the triazole catalyst, MMAE, the organic base and Py-MAA-Val-Cit-PAB-OH is 1-3: 1-3: 1.5-30: 1.

13. The process according to any one of the preceding claims, comprising: after completion of the Reaction B, carrying out pre-HPLC purification to obtain Py-MAA-Val-Cit-PAB-D.

14. The process according to any one of the preceding claims, wherein purification is carried out in Reaction A by the following method to obtain Py-MAA-Val-Cit-PAB-PNP:
(1) centrifuging the reaction solution of Reaction A to obtain a supernatant;
(2) adding solvent 1 to the supernatant, and stirring well;
(3) adding solvent 2 dropwise at 0 to 10 °C, and stirring well;
(4) filtering under suction to obtain a filter cake;
(5) re-dissolving the filter cake in a mixed solution of solvent 3 and solvent 4;
(6) adding the resultant dropwise to solvent 5 at 0 to 10 °C, and stirring;
(7) filtering under suction to obtain an off-white powder, which is purified Py-MAA-Val-Cit-PAB-PNP;
wherein the solvent 1 is a moderately polar solvent, preferably selected from the group consisting of ethyl acetate, dichloromethane, methanol, methyl tert-butyl ether and the like, most preferably ethyl acetate; the solvent 2 is a weakly polar solvent, preferably selected from the group consisting of petroleum ether, n-hexane, n-pentane, cyclohexane and the like, most preferably petroleum ether; the solvent 3 and solvent 4 are solvents in which Py-MAA-Val-Cit-PAB-PNP is easy to dissolve, preferably selected from the group consisting of acetic acid, trifluoroacetic acid, formic acid, methanol, ethanol and the like, most preferably acetic acid and methanol; the solvent 5 is preferably selected from the group consisting of acetonitrile, ethyl acetate, dichloromethane, water and the like, most preferably water.

15. The process according to any one of the preceding claims, wherein the reactions are carried out under the protection of nitrogen.
